# EUROPEAN PATENT APPLICATION

(11) **EP 2 865 674 A1**
(43) Date of publication of application: **29.04.2015**
(21) Application number: 13189944.5
(22) Date of filing: 23.10.2013
(51) Int. Cl.: C07D 305/14

(54) **Crystalline solvate forms of Cabazitaxel**

(71) Applicant: INDENA S.p.A., 20139 Milano (IT)
(72) Inventor: Cabri, Walter, 20139 MILANO (IT); Ciceri, Daniele, 20139 MILANO (IT); Domenighini, Luca, 20139 MILANO (IT); Gambini, Andrea, 20139 MILANO (IT); Peterlongo, Federico, 20139 MILANO (IT)
(74) Representative: Minoja, Fabrizio

(57) **Abstract**

The present invention relates to new crystalline forms of Cabazitaxel of formula (I).

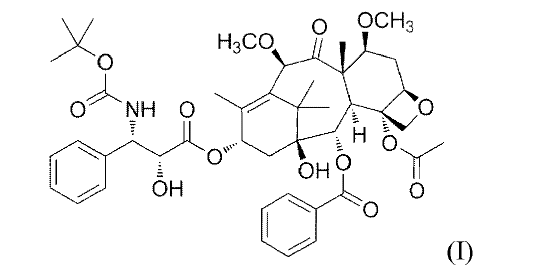

Specifically, the present invention provides four new crystalline solvate forms of Cabazitaxel, designated as form S2 (solvate with 2-methyltetrahydrofuran), form S4 (solvate with *tert*-butyl acetate), form S5 (solvate with dimethylcarbonate) and form S6 (solvate with N-methyl-2-pyrrolidinone).

A further object of the present invention are processes for the preparation of the above mentioned crystalline forms.

The crystalline solvate forms of the invention are especially useful for the preparation of Cabazitaxel, Cabazitaxel salts, and polymorphic forms thereof.

## Description

### FIELD OF THE INVENTION

The present invention relates to new crystalline solvate forms of Cabazitaxel and to processes for the preparation thereof.

### BACKGROUND OF THE INVENTION

Cabazitaxel is a semi-synthetic derivative of the natural taxoid 10-deacetylbaccatin III, commercialized as acetone solvate. It stabilizes microtubules leading eventually to the mitotic arrest of proliferating cells. It has been approved in the United States of America for the second line treatment of hormone-refractory prostate cancer following a docetaxel-based treatment.

Cabazitaxel has the following formula (I):

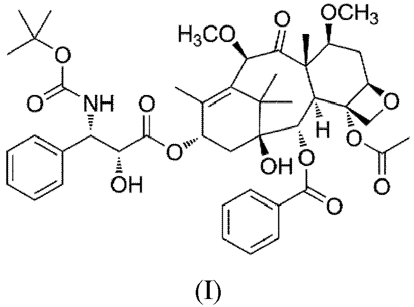

Its chemical name is 4α-acetoxy-2α-benzoyloxy-5β,20-epoxy-1β-hydroxy-7β,10β-dimethoxy-9-oxo-11-taxen-13α-yl (2R,3S)-3-*tert*-butoxycarbonylamino-2-hydroxy-3-phenylpropionate.

Cabazitaxel and methods for the preparation thereof are described in WO96/30355 and in WO99/25704.

WO2005/028462 describes an acetone solvate of Cabazitaxel, sometimes referred to as form A.

Additional crystalline solvate forms of Cabazitaxel referred to as form I (toluene solvate), form II (methyl *tert*-butyl ether solvate), form III (2-propanol solvate), form IV (1-butanol solvate), form V (1-propanol solvate) and an amorphous form of Cabazitaxel in a powdery, non-foamy form are described in WO2012/142117 (Teva). Solvates are rarely used in pharmaceuticals because the solvents are volatile thus making it difficult to maintain the solvent in the crystal. If the API desolvates due to storage conditions or otherwise, it could lead to the formation of multiple polymorphs with different physical properties. Additionally, amorphous solids are metastable and can lead with time to the formation of different polymorphs with different physical properties.

WO2009/115655 (Sanofi) discloses five anhydrous forms of the compound, referred to as forms B, C, D, E and F; three ethanol solvates, referred to as ethanolate forms B, D, E; an ethanol/water heterosolvate form F; and a monohydrate-solvent free form C and a dihydrate-solvent free form C. Reaching high purities with these forms is only possible providing the API has been previously purified by other techniques such as for example passing through the acetone solvate (as described in the application). However the introduction of a further purification technique hampers the manufacturing process with inefficiency due to longer production times and lower yield.

WO 2013/134534 discloses crystalline Cabazitaxel solvates with:
- alkyl acetates, such us the solvates with ethyl acetate (Form VII), isopropyl acetate (Form VIII), methyl acetate (Form XVII), butyl acetate (Form XVIII) and isobutyl acetate (Form XXI);
- ketones, such as the solvates with methyl ethyl ketone (Form IX) and methyl isobutyl ketone (Form X);
- alcohols, such as the solvates with 2-butanol (Form XI), isobutanol (Form XII) and amyl alcohol (Form XIII).

WO 2013/134534 also describes solvates with dioxolane (Form XIV), 1,4-dioxane (Form XV), 1,2-propanediol (Form XIX), glycerol (Form XX) and 1,3-dimethy-2-imidazolidinone (Form XXII). A crystalline cabazitaxel form designated as Form XVI, which may be anhydrous, is also disclosed.

A crystalline ethyl acetate solvate of Cabazitaxel is disclosed also in WO 2013/088335.

WO2009/115655 discloses two hydrate forms of the compound in particular mono and di-hydrate, both hydrate forms are obtained from anhydrous form C by exposition to moisture. The anhydrous form C as described above is obtained in high purity only by passing through the acetone solvate.

A crystalline form of Cabazitaxel obtained from acetone/water is described in CN 102675257 A.

Crystalline forms, including an anhydrate form, of Cabazitaxel, designated as Forms C1, C2, C3, C4, C5, C6, C7, C8, C8b, C9 and C9p are described in WO2013/034979.

Finally, 13 crystalline forms referred to as Form-1, Form-2, Form-3, Form-4, Form-5, Form-6, Form-7, Form-8, Form-9, Form-10, Form-11, Form-12, and Form-13 are disclosed in WO2013/0109870.

It is still desirable to find new crystalline forms able to solve the aforementioned problems.

### BRIEF DESCRIPTION OF THE INVENTION

Object of the present invention are four new crystalline solvate forms of Cabazitaxel, designated as form S2, form S4, form S5 and form S6. A further object of the present invention are processes for the preparation of the above mentioned new crystalline forms.

In the present invention the term "solvate" refers to a crystalline form of Cabazitaxel that incorporates a solvent in the crystal structure, in either a stoichiometric or in a non-stoichiometric amount.

Form S2 is a Cabazitaxel solvate with 2-methyltetrahydrofuran (MHTF), obtained by crystallization of Cabazitaxel from a MHTF/n-hexane mixture. In the crystalline form the Cabazitaxel/MHTF molar ratio is about 1:0.8.

Form S4 is a Cabazitaxel solvate with *tert*-butyl acetate (*t*BuOAc) obtained by crystallization of Cabazitaxel from *t*BuOAc. In the crystalline form the Cabazitaxel/*t*BuOAc molar ratio is about 1:1.

Form S5 is a Cabazitaxel solvate with dimethylcarbonate (DMC) obtained by crystallization of Cabazitaxel from DMC. In the crystalline form the Cabazitaxel/DMC molar ratio is about 1:0.25.

Form S6 is a Cabazitaxel solvate with N-methyl-2-pyrrolidinone (NMP) obtained by crystallization of Cabazitaxel from a NMP/water mixture. In the crystalline form the Cabazitaxel/NMP molar ratio is about 1:0.9.

### BRIEF DESCRIPTION OF THE FIGURES

Figure 1 X-RPD pattern of the crystalline form S2 of Cabazitaxel
Figure 2 FTIR spectrum of the crystalline form S2 of Cabazitaxel in the 4000-550 cm⁻¹ spectral range
Figure 3 TG and DTA profiles of the crystalline form S2 of Cabazitaxel
Figure 4 X-RPD pattern of the crystalline form S4 of Cabazitaxel
Figure 5 FTIR spectrum of the crystalline form S4 of Cabazitaxel in the 4000-550 cm⁻¹ spectral range
Figure 6 TG and DTA profiles of the crystalline form S4 of Cabazitaxel
Figure 7 X-RPD pattern of the crystalline form S5 of Cabazitaxel
Figure 8 FTIR spectrum of the crystalline form S5 of Cabazitaxel in the 4000-550 cm⁻¹ spectral range
Figure 9 TG and DTA profiles of the crystalline form S5 of Cabazitaxel
Figure 10 X-RPD pattern of the crystalline form S6 of Cabazitaxel
Figure 11 FTIR spectrum of the crystalline form S6 of Cabazitaxel in the 4000-550 cm⁻¹ spectral range
Figure 12 TG and DTA profiles of the crystalline form S6 of Cabazitaxel

### DETAILED DESCRIPTION OF THE INVENTION

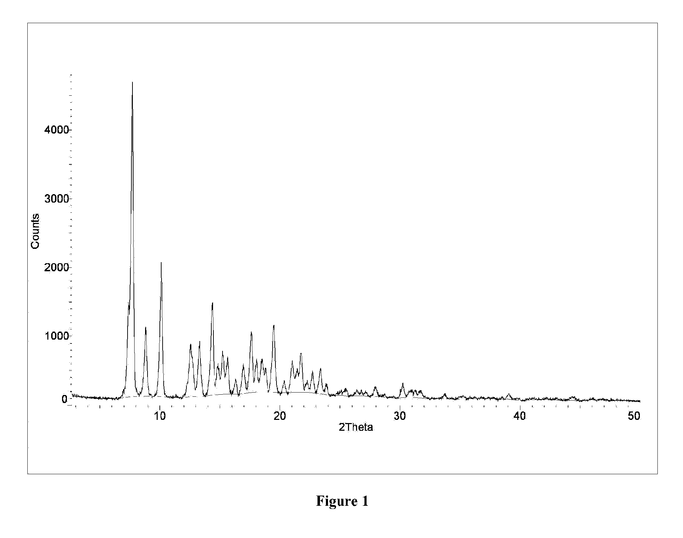

Form S2 of Cabazitaxel according to the present invention is characterised by a X-Ray Powder Diffraction (X-RPD) pattern obtained using the copper wavelengths λ₁ and λ₂ of 1.54056 Å and 1.54439 Å, respectively, essentially as depicted in Figure 1. The X-RPD pattern shows a crystalline structure and comprises distinctive reflections, expressed as 2-theta degrees values, at 7.4, 7.7, 8.8, 10.1, 12.6, 13.3, 14.4, 14.8, 15.2, 15.6, 16.3, 17.0, 17.6, 18.0, 18.5, 18.8 and 19.5 ± 0.2.

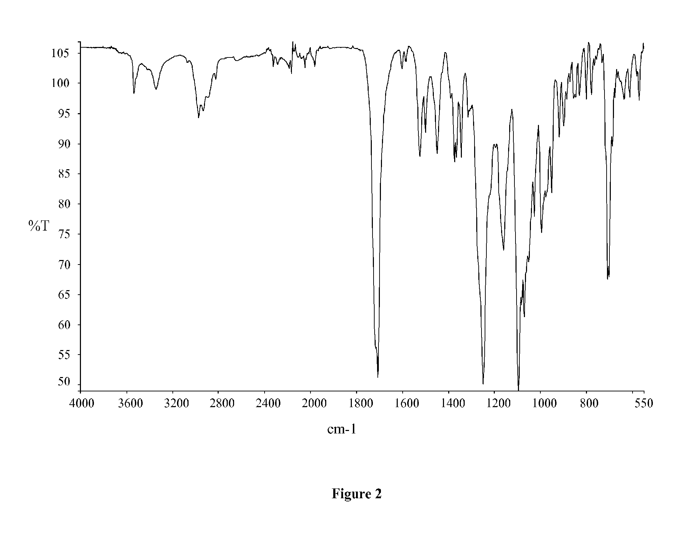

Form S2 may be further characterised by a Fourier-Transform InfraRed Spectroscopy (FTIR) spectrum acquired in the 4000-550 cm⁻¹ spectral range in ATR mode, essentially as depicted in Figure 2. The FTIR spectrum of form S2 comprises characteristic absorption frequencies at 3536, 3343, 2973, 2934, 2825, 1707, 1525, 1500, 1450, 1375, 1366, 1345, 1250, 1161, 1096, 1071, 997, 952, 919, 831, 801, 709, 703 and 687 ± 4 cm⁻¹.

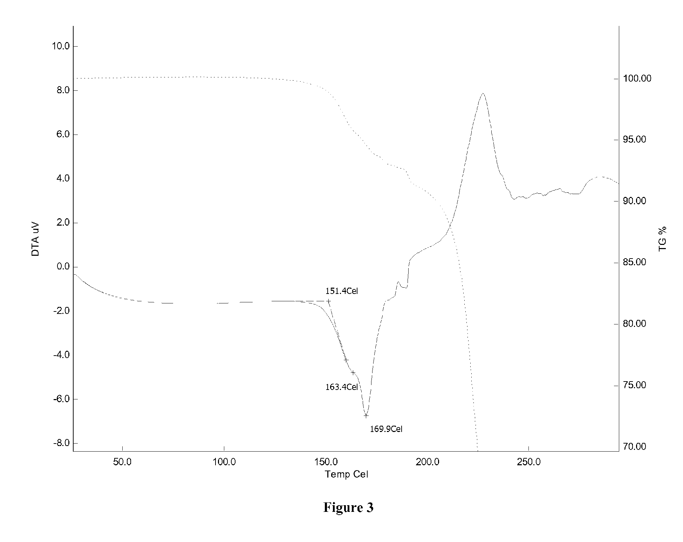

Form S2 may be further characterised by Thermogravimetric (TG) and Differential Thermal Analysis (DTA) profiles, essentially as depicted in Figure 3. The DTA profile is characterised by a non-resolved endothermic peak with onset at about 151°C and maximum respectively at 163.4°C and 169.9°C.

The solvent content of form S2 has been determined by ¹H-NMR and the obtained value is about 7.4% of MTHF and 0.17% of *n*-hexane by weight and the Cabazitaxel/MHTF mole/mole ratio is about 1:0.8.

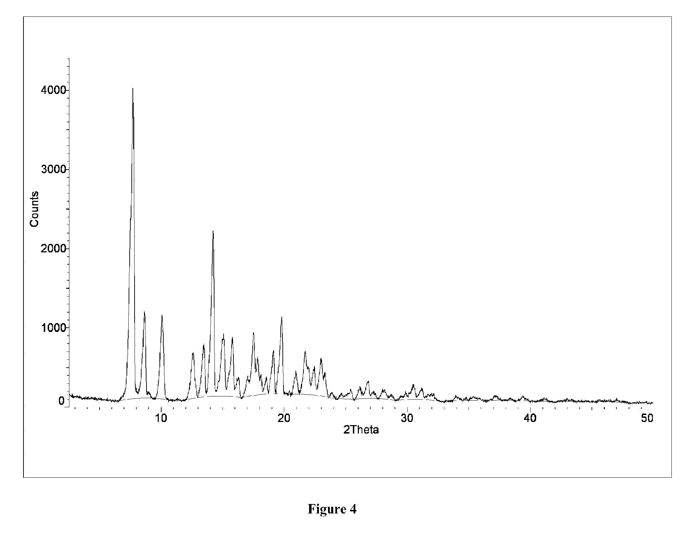

Form S4 of Cabazitaxel according to the present invention is characterised by a X-Ray Powder Diffraction (X-RPD) pattern obtained using the copper wavelengths λ₁ and λ₂ of 1.54056 Å and 1.54439 Å, respectively, essentially as depicted in Figure 4. The X-RPD pattern shows a crystalline structure and comprises distinctive reflections, expressed as 2-theta degrees values, at 7.7, 8.6, 10.1, 12.6, 13.5, 14.2, 15.0, 15.8, 16.2, 17.1, 17.5, 17.8, 18.1, 18.5, 19.1 and 19.8 ± 0.2.

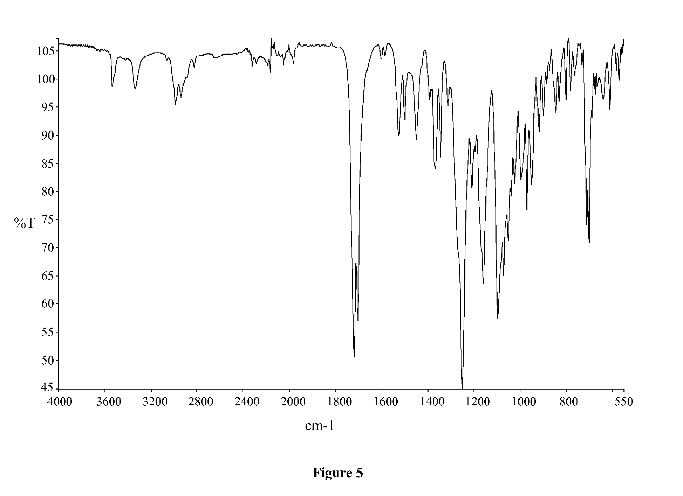

Form S4 may be further characterised by a Fourier-Transform InfraRed Spectroscopy (FTIR) spectrum, acquired in the 4000-550 cm⁻¹ spectral range in ATR mode, essentially as depicted in Figure 5. The FTIR spectrum of form S4 comprises characteristic absorption frequencies at 3536, 3337, 2988, 2940, 2826, 1719, 1703, 1525, 1500, 1449, 1392,1365, 1344, 1251, 1209, 1159, 1097, 1071, 1052, 997, 971, 950, 917, 899, 846, 831, 801, 782, 765, 711, 702 and 611 ± 4 cm⁻¹.

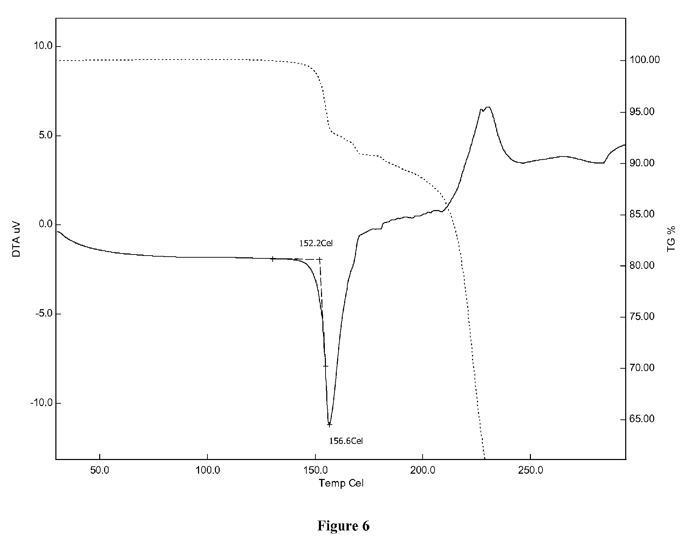

Form S4 may be further characterised by Thermogravimetric (TG) and Differential Thermal Analysis (DTA) profiles, essentially as depicted in Figure 6. The DTA profile is characterised by an endothermic peak with onset at about 152°C and maximum at 156.6°C.

The solvent content of form S4 has been determined by ¹H-NMR and the obtained value is about 10.9% by weight of *t*BuOAc and the Cabazitaxel/ *t*BuOAc mole/mole ratio is about 1: 0.9.

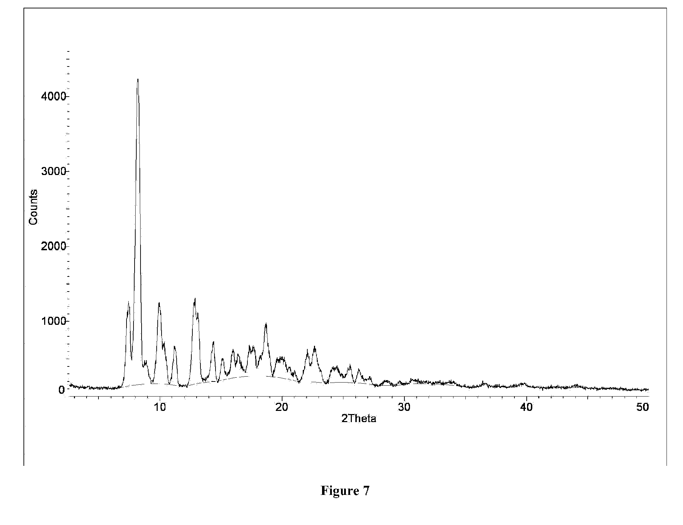

Form S5 of Cabazitaxel according to the present invention is characterised by a X-Ray Powder Diffraction (X-RPD) pattern obtained using the copper wavelengths λ₁ and λ₂ of 1.54056 Å and 1.54439 Å, respectively, essentially as depicted in Figure 7. The X-RPD pattern shows a crystalline structure and comprises distinctive reflections, expressed as 2-theta degrees values, at 7.4, 8.2, 8.8, 10.0, 10.3, 11.2, 12.8, 13.0, 14.4, 15.1, 16.0, 16.4, 17.4, 17.6 and 18.7 ± 0.2.

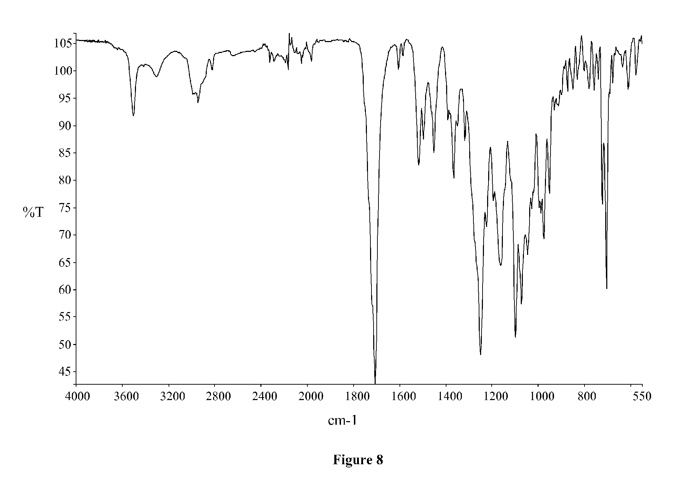

Form S5 may be further characterised by a Fourier-Transform InfraRed Spectroscopy (FTIR) spectrum, acquired in the 4000-550 cm⁻¹ spectral range in ATR mode, essentially as depicted in Figure 8. The FTIR spectrum of form S5 comprises characteristic absorption frequencies at 3505, 3307, 2946, 2822, 1706, 1517, 1497, 1451, 1366, 1317, 1250, 1163, 1099, 1072, 1046, 988, 976, 952, 874, 850, 831, 780, 758, 741, 723, 705 and 610 ± 4 cm⁻¹.

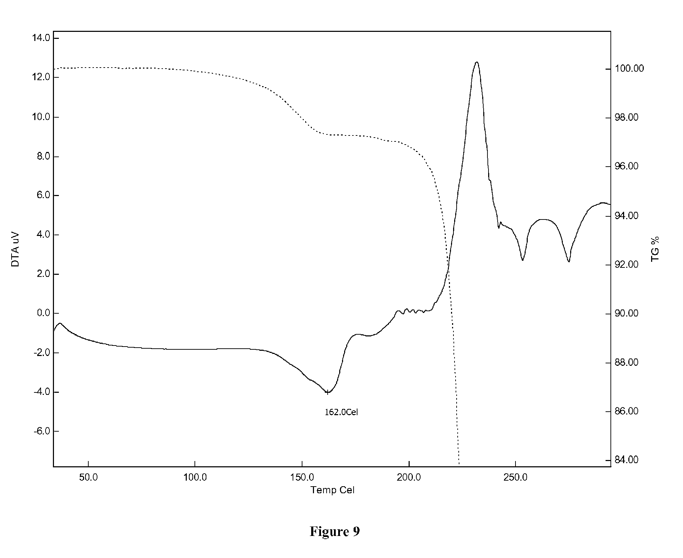

Form S5 may be further characterised by Thermogravimetric (TG) and Differential Thermal Analysis (DTA) profiles, essentially as depicted in Figure 9. The DTA profile is characterised by a broad endothermic signal with maximum at 162.0°C.

The solvent content of form S5 has been determined by ¹H-NMR and the obtained value is about 2.5% by weight of DMC and the Cabazitaxel/DMC mole/mole ratio is about 1:0.25.

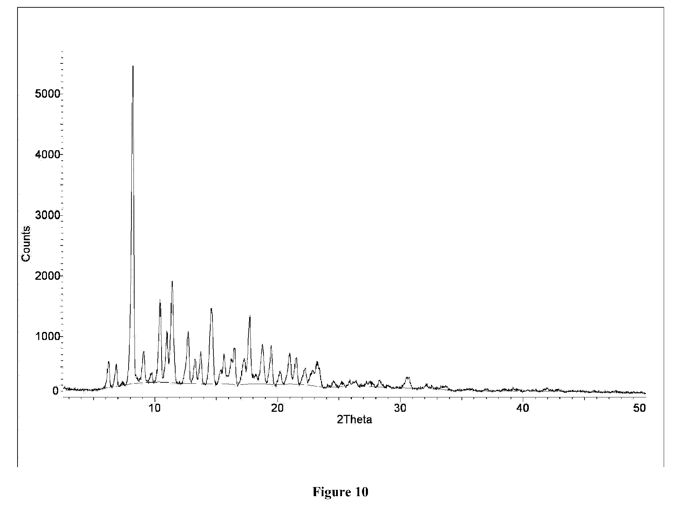

Form S6 of Cabazitaxel according to the present invention is characterised by a X-Ray Powder Diffraction (X-RPD) pattern obtained using the copper wavelengths λ₁ and λ₂ of 1.54056 Å and 1.54439 Å, respectively, essentially as depicted in Figure 10. The X-RPD pattern shows a crystalline structure and comprises distinctive reflections, expressed as 2-theta degrees values, at 6.2, 6.8, 7.4, 8.2, 9.1, 9.7, 10.4, 11.0, 11.4, 12.7, 13.3, 13.7, 14.6, 15.4, 15.6, 16.3, 16.5, 17.3, 17.7, 18.2, 18.8 and 19.5 ± 0.2.

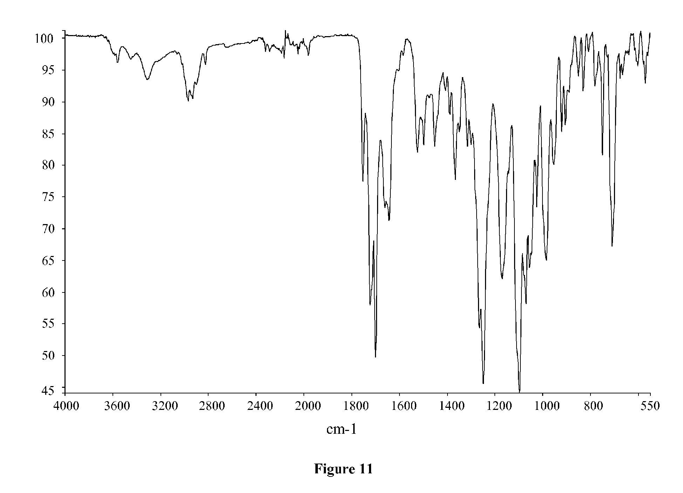

Form S6 may be further characterised by a Fourier-Transform InfraRed Spectroscopy (FTIR) spectrum, acquired in the 4000-550 cm⁻¹ spectral range in ATR mode, essentially as depicted in Figure 11. The FTIR spectrum of form S6 comprises characteristic absorption frequencies at 3562, 3449, 3313, 2969, 2933, 2825, 1753, 1723, 1700, 1661, 1644, 1525, 1498, 1453, 1367, 1266, 1250, 1172, 1098, 1071, 1026, 987, 955, 922, 906, 832, 751, 711 and 602 ± 4 cm⁻¹.

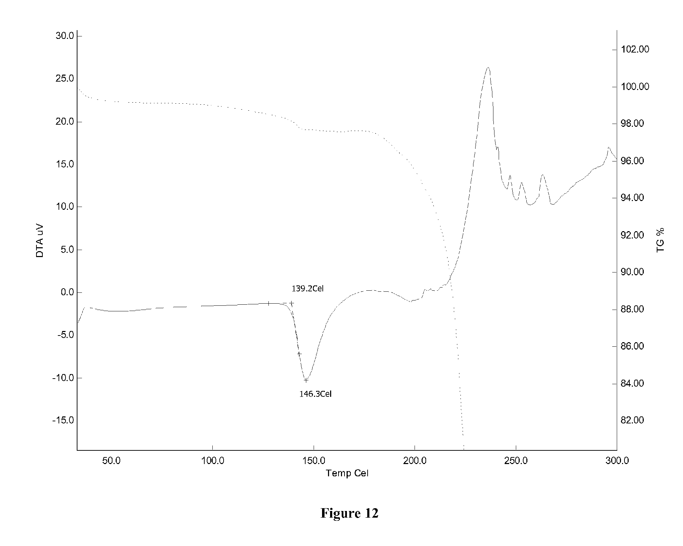

Form S6 may be further characterised by Thermogravimetric (TG) and Differential Thermal Analysis (DTA) profiles, essentially as depicted in Figure 12. The DTA profile is characterised by an endothermic peak with onset at about 139°C and maximum at 146.3°C. The solvent content of form S6 has been determined by ¹H-NMR and the obtained value is about 9.2% by weight of NMP and the Cabazitaxel/NMP mole/mole ratio is about 1:0.9.

When the crystalline solvate forms of Cabazitaxel according to the present invention are referred to herein as being characterized by graphical data essentially as depicted in a figure, such as for, for example, the X-RPD diffractogram, the TG/DTA and DSC profiles, the FTIR spectrum, the skilled person will understand that such graphical representations of data may be affected by small variations which may be triggered by experimental variability affecting the instrumental response and/or the sample concentration and purity. These variations are well known to the skilled person and they will not prevent him from comparing the graphical data in the figures herein with graphical data generated for an unknown crystal form and from assessing whether the two sets of graphical data are characterizing the same crystal form or two different crystal forms.

In general, the crystalline solvate forms of the present invention may be prepared by a process comprising the steps of dissolving Cabazitaxel in an organic solvent selected form MTHF, *t*BuOAc, DMC or NMP, optionally by heating. Precipitation of the crystals of the *t*BuOAC or DMC solvate typically occurs spontaneously upon stirring at room temperature, whereas in the case of the MTHF or NMP solvate it may be induced by the addition of an anti-solvent such as hexane or water, respectively.

The crystalline solvate form S2 may be prepared by recrystallization of crude Cabazitaxel from a mixture of MHTF as described in Example 1.

A further object of the present invention is therefore a process for the preparation of the crystalline solvate form S2 of Cabazitaxel comprising the following steps:
a) dissolution of Cabazitaxel in MHTF at room temperature;
b) addition of n-hexane to the solution obtained in step a), wherein a product starts to crystallize;
c) filtration and drying of the precipitate obtained in step b), to afford the crystalline solvate form S2 of Cabazitaxel.

In one embodiment, in step b) the addition of n-hexane is performed drop-wise, using one volume of n-hexane for one volume of MHTF and the slurry was stirred at room temperature.

The crystalline solvate form S4 may be prepared by recrystallization of crude Cabazitaxel from *t*BuOAc as described in Example 2.

A further object of the invention is therefore a process for the preparation of the crystalline solvate form S4 of Cabazitaxel comprising the following steps:
a) dissolution of Cabazitaxel at temperature higher than 30°C in *t*BuOAc;
b) cooling of the solution obtained in step a) to room temperature, wherein a product starts to crystallize;
c) filtration and drying of the precipitate obtained in step b), to afford the crystalline solvate form S4 of Cabazitaxel.

The crystalline solvate form S5 may be prepared by recrystallization of crude Cabazitaxel from DMC as described in Example 3.

A further object of the invention is therefore a process for the preparation of the crystalline solvate form S5 of Cabazitaxel comprising the following steps:
a) dissolution of Cabazitaxel at temperature higher than 30°C in DMC;
b) cooling of the solution obtained in step a) to room temperature, wherein a product starts to crystallize;
c) filtration and drying of the precipitate obtained in step b), to afford the crystalline solvate form S5 of Cabazitaxel.

The crystalline solvate form S6 may be prepared by recrystallization of crude Cabazitaxel form a mixture of NMP and water as described in Example 4.

A further object of the invention is therefore a process for the preparation of the crystalline solvate form S6 of Cabazitaxel comprising the following steps:
a) dissolution of Cabazitaxel in NMP at temperature higher than 30°C;
b) adding water to the solution obtained in step a), to obtain a slurry;
c) cooling the slurry obtained in step b) to room temperature;
d) filtration and drying of the precipitate obtained in step c), to afford the crystalline solvate form S6 of Cabazitaxel.

In one embodiment, in step b) the addition of water is performed drop-wise, using one volume of water for one volume of NMP.

The crystalline forms of the invention may be obtained with purity higher than 98% when obtained as described in the examples 1-4 starting from crude Cabazitaxel. The solvate forms S5 and S6 are obtained typically with purity higher than 99%.

The crystalline solvate forms of the invention are endowed with several advantageous properties as compared to the previously disclosed forms of Cabazitaxel in term of, for example, high purity obtainable without the need of an additional crystallisation, stability to conversion to other polymorphic forms, better handling and improved processability.

In view of the above described advantages, the crystalline solvate forms of Cabazitaxel of the invention are especially useful for the preparation of Cabazitaxel, Cabazitaxel salts, and polymorphic forms thereof.

The invention is now further illustrated by the following examples, wherein a crude Cabazitaxel was used as starting material.

### EXAMPLE 1

### Preparation of Cabazitaxel solvate form S2 by 2-methyl-tetrahydrofuran (MTHF)/hexane crystallization of crude Cabazitaxel

Crude Cabazitaxel (1 g) was dissolved in MHTF (10 mL) at room temperature and hexane (10 mL) was added dropwise. The product starts to crystallize and the slurry was filtered, washed and dried under vacuum at about 60°C for 16 hours. Cabazitaxel solvate form S2 (0.65 g) with purity higher than 98% was obtained. Yield 65%.

### EXAMPLE 2

### Preparation of Cabazitaxel solvate form S4 by tert-butyl acetate (tBuOAc) crystallization of crude Cabazitaxel

Crude Cabazitaxel (1 g) was dissolved in *t*BuOAc (60 mL) at temperature higher than 30°C. Subsequent to complete dissolution, the temperature was decreased to room temperature and the product was left to crystallize. The precipitate was filtered, washed and dried under vacuum for 16 hours at about 60°C. Cabazitaxel solvate form S4 (0.90 g) with purity higher than 98% was obtained. Yield 90%.

### EXAMPLE 3

### Preparation of Cabazitaxel solvate form S5 by dimethyl carbonate (DMC) crystallization of crude Cabazitaxel

Crude Cabazitaxel (1 g) was dissolved in DMC (6 mL) at temperature higher than 30°C. Subsequent to complete dissolution, the temperature was decreased to room temperature and the product was left to crystallize. The precipitate was filtered, washed and dried under vacuum for 16 hours at about 60°C. Cabazitaxel solvate form S5 (0.85 g) with all impurities below 0.10% (HPLC analysis) was obtained. Yield 85%.

### EXAMPLE 4

### Preparation of Cabazitaxel solvate form S6 by N-methyl-2-pyrrolidinone (NMP)/water crystallization of crude Cabazitaxel

Crude Cabazitaxel (1 g) was dissolved in NMP (10 mL) at temperature higher than 30°C. Subsequent to complete dissolution, water was added dropwise and the slurry was cooled to room temperature. The precipitate was filtered, washed and dried under vacuum for 16 hours at about 60°C. Cabazitaxel solvate form S6 (0.90 g) with all impurities below 0.10% (HPLC analysis) was obtained. Yield 90%.

### EXAMPLE 5

The compounds obtained according to Examples 1-4 were characterized using the below described techniques.

### X-Ray Powder Diffraction (X-RPD)

X-RPD patterns were collected on a Bruker D2-Phaser Diffractometer. The x-ray generator was operated at 30kV and 10 mA, using the CuKα line as the radiation source. The sample was packed on a suitable slit and the irradiated length was 10mm. Data were collected between 2 and 50 deg 2-theta with a step size of 0.02 deg 2-theta and a counting time per step of 3 sec.

### Thermogravimetry (TG) and Differential Thermal Analysis (DTA)

The analysis was performed using a Seiko TG/DTA7200 simultaneous system using open aluminum pans (40 µl volume). The TG/DT signals were recorded from 30 to 300°C with linear heating rate (10°C/min) under a 200 ml/min nitrogen flow. About 10 mg of powder was used for the measurement.

### Solvent content

The solvent content was determined by ¹H-NMR using a Varian 300 MHz instrument.

### Fourier-Transform InfraRed Spectroscopy (FTIR)

The infrared spectrum was recorded in Attenuated Total Reflectance (ATR) mode using Fourier-Transform spectrometer Perkin Elmer Spectrum One, equipped with Specac ATR Golden Gate accessory. The spectrum is the result of the acquisition and transformation of 16 co-added scans in the 4000-550 cm⁻¹ spectral region at a resolution of 4 cm⁻¹.

## Claims

1. A crystalline solvate form of Cabazitaxel of formula (I) 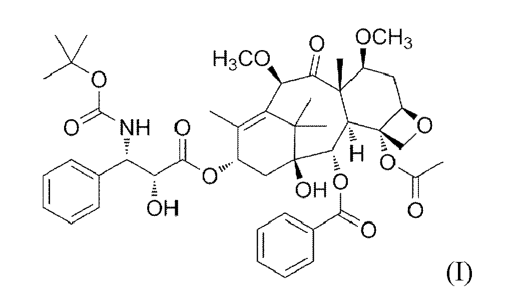 with 2-methyltetrahydrofuran.

2. The crystalline solvate form according to claim 1, referred to as form S2, having one or more of the following:
- an X-RPD pattern, obtained using the copper wavelengths λ₁ and λ₂ of 1.54056 Å and 1.54439 Å, respectively, comprising distinctive reflections, expressed as 2-theta degrees values, at 7.4, 7.7, 8.8, 10.1, 12.6, 13.3, 14.4, 14.8, 15.2, 15.6, 16.3, 17.0, 17.6, 18.0, 18.5, 18.8 and 19.5 ± 0.2;
- an XRPD pattern obtained using the copper wavelengths λ₁ and λ₂ of 1.54056 Å and 1.54439 Å, respectively, essentially as depicted in Figure 1;
- TG and DTA profiles obtained with a linear heating rate of 10°C/min essentially as depicted in Figure 3.

3. A crystalline solvate form of Cabazitaxel of formula (I) 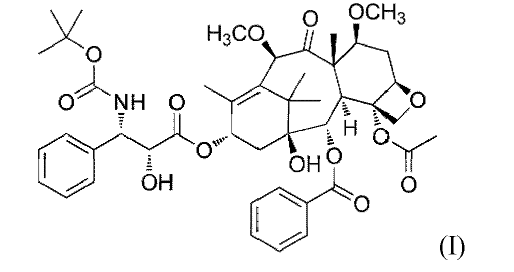 with *tert*-butylacetate.

4. The crystalline solvate form according to claim 3, referred to as form S4, having one or more of the following:
- an XRPD pattern obtained using the copper wavelengths λ₁ and λ₂ of 1.54056 Å and 1.54439 Å, respectively, comprising distinctive reflections, expressed as 2-theta degrees values, at 7.7, 8.6, 10.1, 12.6, 13.5, 14.2, 15.0, 15.8, 16.2, 17.1, 17.5, 17.8, 18.1, 18.5, 19.1 and 19.8 ± 0.2;
- an XRPD pattern, obtained using the copper wavelengths λ₁ and λ₂ of 1.54056 Å and 1.54439 Å, respectively, essentially as depicted in Figure 4;
- TG and DTA profiles obtained with a linear heating rate of 10°C/min essentially as depicted in Figure 6.

5. A crystalline solvate form of Cabazitaxel of formula (I) 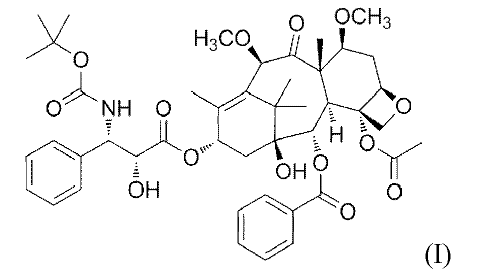 with dimethylcarbonate.

6. The crystalline solvate form of claim 5, referred to us form S5, having one or more of the following:
- an X-RPD pattern, obtained using the copper wavelengths λ₁ and λ₂ of 1.54056 Å and 1.54439 Å, respectively, comprising distinctive reflections, expressed as 2-theta degrees values, at 7.4, 8.2, 8.8, 10.0, 10.3, 11.2, 12.8, 13.0, 14.4, 15.1, 16.0, 16.4, 17.4, 17.6 and 18.7 ± 0.2;
- an X-RPD pattern obtained using the copper wavelengths λ₁ and λ₂ of 1.54056 Å and 1.54439 Å, respectively, essentially as depicted in Figure 7;
- TG and DTA profiles obtained with a linear heating rate of 10°C/min essentially as depicted in Figure 9.

7. A crystalline solvate form of Cabazitaxel of formula (I) 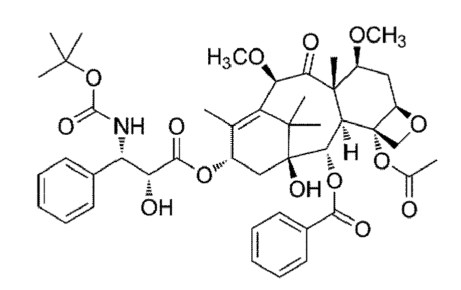 with N-methyl-2-pyrrolidinone.

8. The crystalline form of claim 7, referred to as form S6, having one or more of the following:
- an X-RPD pattern, obtained using the copper wavelengths λ₁ and λ₂ of 1.54056 Å and 1.54439 Å, respectively, comprising distinctive reflections, expressed as 2-theta degrees values, at 6.2, 6.8, 7.4, 8.2, 9.1, 9.7, 10.4, 11.0, 11.4, 12.7, 13.3, 13.7, 14.6, 15.4, 15.6, 16.3, 16.5, 17.3, 17.7, 18.2, 18.8 and 19.5 ± 0.2;
- an X-RPD pattern, obtained using the copper wavelengths λ₁ and λ₂ of 1.54056 Å and 1.54439 Å, respectively, essentially as depicted in Figure 10;
- TG and DTA profiles obtained with a linear heating rate of 10°C/min essentially as depicted in Figure 12;

9. The crystalline forms of Cabazitaxel according to claims 1-8, for use in the preparation of Cabazitaxel, Cabazitaxel salts and polymorphic forms thereof.

10. A process for the preparation of the crystalline solvate form of Cabazitaxel according to claims 1-2, comprising the crystallization of Cabazitaxel from a mixture of 2-methytetrahydrofuran and hexane.

11. A process for the preparation of the crystalline solvate form of Cabazitaxel according to claims 3-4, comprising the crystallization of Cabazitaxel from *tert*-butylacetate.

12. A process for the preparation of the crystalline solvate form of Cabazitaxel according to claims 5-6, comprising the crystallization of Cabazitaxel from dimethylcarbonate.

13. A process for the preparation of the crystalline solvate form of Cabazitaxel according to claims 7-8, comprising the crystallization of Cabazitaxel from a mixture of N-methyl-2-pyrrolidinone and water.
